(19) Europäisches Patentamt
European Patent Office
Office européen des brevets

(11) Publication number: **0 383 941 B1**

(12) **EUROPEAN PATENT SPECIFICATION**

(45) Date of publication of patent specification: **03.11.93**

(51) Int. Cl.5: **A61K 7/42**

(21) Application number: **89909430.4**

(22) Date of filing: **18.08.89**

(86) International application number:
**PCT/JP89/00842**

(87) International publication number:
**WO 90/01924 (08.03.90 90/06)**

(54) **ULTRAVIOLET ABSORBING COSMETIC.**

(30) Priority: **19.08.88 JP 204596/88**

(43) Date of publication of application:
**29.08.90 Bulletin 90/35**

(45) Publication of the grant of the patent:
**03.11.93 Bulletin 93/44**

(84) Designated Contracting States:
**CH DE FR GB IT LI**

(56) References cited:
**FR-A- 2 301 524**
**JP-A- 5 912 952**
**JP-A- 6 211 744**
**JP-A-57 209 979**

**PATENT ABSTRACTS OF JAPAN, vol. 11, no. 163 (C-424)[2610], 26th May 1987; & JP-A-61 291 575**

(73) Proprietor: **TEIJIN LIMITED**
**6-7, Minamihonmachi 1-chome**
**Chuo-ku**
**Osaka-shi Osaka 541(JP)**

Proprietor: **TAKEMOTO OIL & FAT CO., LTD.**
**2-5, Minato-machi, Gamagori-shi**
**Aichi 443(JP)**

(72) Inventor: **YOSHIDA, Norio**
**4-11-26, Shimohozumi**
**Ibaraki-shi Osaka 567(JP)**
Inventor: **SUGIURA, Masato**
**955-1, Arimae**
**Fuso-cho,**
**Gamagori-shi Aichi 443(JP)**
Inventor: **SUGIURA, Fumitoshi**
**26-5, Sakuma**
**Takenoya-cho**
**Gamagori-shi Aichi 443(JP)**

**EP 0 383 941 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(74) Representative: **Cresswell, Thomas Anthony et al**
**J.A. Kemp & Co.**
**14 South Square**
**Gray's Inn**
**London WC1R 5LX (GB)**

## Description

This invention relates to a cosmetic containing a particular cyclic imino ester as an ultraviolet (UV) absorber.

Although sunbathing is frequently performed, erythema results from excessive exposure of the skin to sunlight, this phenomenon being caused mainly by radiation having a wavelength of 280 to 320 nm, so-called UV-B. To prevent such burning it has been proposed to use an ultraviolet absorber having absorption in the region of UV-B (see JP-B-39404/1984 and the corresponding US-A-4,061,730).

It has been thought that radiation having a wavelength of 320 to 400 nm (UV-A), which makes the skin brown without causing erythema, is desirable. However, although UV-B penetrates only the epithelial layer, UV-A penetrates further and into the underlying dermal layer, so that the skin is aged by long-term exposure to UV-A.

Therefore, cosmetics containing a compound having an absorption band in the region of UV-A have been eagerly sought, and substituted dibenzoylmethanes have been proposed as compounds having such effects (see JP-B-4813/1986 and US-A-4,489,057). However, substituted dibenzoylmethanes do not exhibit sufficient effect since their absorption ability rapidly decreases at 350 nm or more. Further, their water resistance is not sufficient.

US-A-4,446,262 discloses a light-stabilized polymer composition containing as an ultraviolet absorber a compound of formula (I)

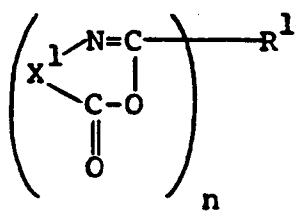

wherein $X^1$ is 1,2-phenylene, 1,2-naphthylene, 2,3-naphthylene, a group of formula (a)

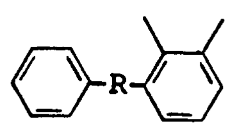

(wherein, R is -O-, -CO-, -S-, $-SO_2-$, $CH_2-$ or $-(CH_2)_2-$ or

$$-C(CH_3)_2-,$$

or a group of formula (b)

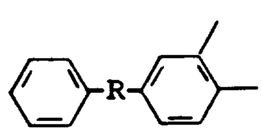

(wherein, R is as defined above);
n is 1, 2 or 3; and $R^1$ is a n-valent hydrocarbon. This composition seeks to protect against deterioration due to exposure to UV the polymer base material itself, for example, a thermoplastic resin such as polyester, polyamide, polycarbonate, polyolefin, polyether or polysulfone, or a thermosetting resin such as phenolformaldehyde resin, melamine resin, polyurethane resin, urea resin, epoxy resin or unsaturated polyester resin. There is no disclosure relating to cosmetics.

According to the invention there is provided a cosmetic containing a cyclic imino ester of formula (I)

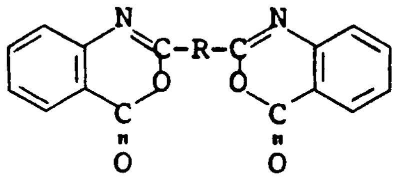

. . . (I)

(wherein R is a divalent aromatic hydrocarbon residue) in a quantity effective for the absorption of ultraviolet radiation, together with cosmetic base.

In the above formula (I) R is a divalent aromatic hydrocarbon residue. Preferred examples of these aromatic hydrocarbon residues include phenylene, biphenylene and naphthylene. The position of the free bonds of these aromatic hydrocarbon groups is optional. For example, there can be mentioned as more specific examples o-, m- or p-phenylene, 4,4'-biphenylene, 3,3'-biphenylene, 1,5-naphthylene and 2,6-naphthylene.

Further, these aromatic hydrocarbon groups can have a substituent. Examples of such substituents include alkyl groups having 1 to 4 carbon atoms, nitro group and halogen atom. The alkyl groups having 1 to 4 carbon atoms may be either straight chain or branched chain, and can be any of methyl, ethyl, n-propyl, isopropyl, n-butyl, isobutyl, sec-butyl and t-butyl. Among them methyl is particularly preferred. Examples of halogen include fluoro, chloro and bromo. Among them chloro is particularly preferred.

Examples of the cyclic imino esters of formula (I) include
2,2'-p-phenylenebis(3,1-benzoxazin-4-one),
2,2'-(4,4'-biphenylene)bis(3,1-benzoxazin-4-one),
2,2'-(2,6-naphthylene)bis(3,1-benzoxazin-4-one),
2,2'-(1,5 -naphthylene)bis(3,1-benzoxazin-4-one),
2,2'-(2-methyl-p-phenylene)bis(3,1-benzoxazin-4-one),
2,2'-(2-nitro-p-phenylene)bis(3,1-benzoxazin-4-one) and
2,2'-(2-chloro-p-phenylene)bis(3,1-benzoxazin-4-one).

These compounds can be prepared according to processes per se known.

For example, there can be used a process which comprises reacting anthranilic acid with an aromatic dicarboxylic acid dichloride according to the process disclosed in US-A-3,408,326 to form an N,N'-bis-2-carboxyphenyl-substituted aromatic dicarboxylic acid diamide, and then heating the diamide together with acetic anhydride to dehydrate and cyclize it.

This process can be represented by the following reaction formula when using terephthalic acid dichloride as the aromatic dicarboxylic acid dichloride:

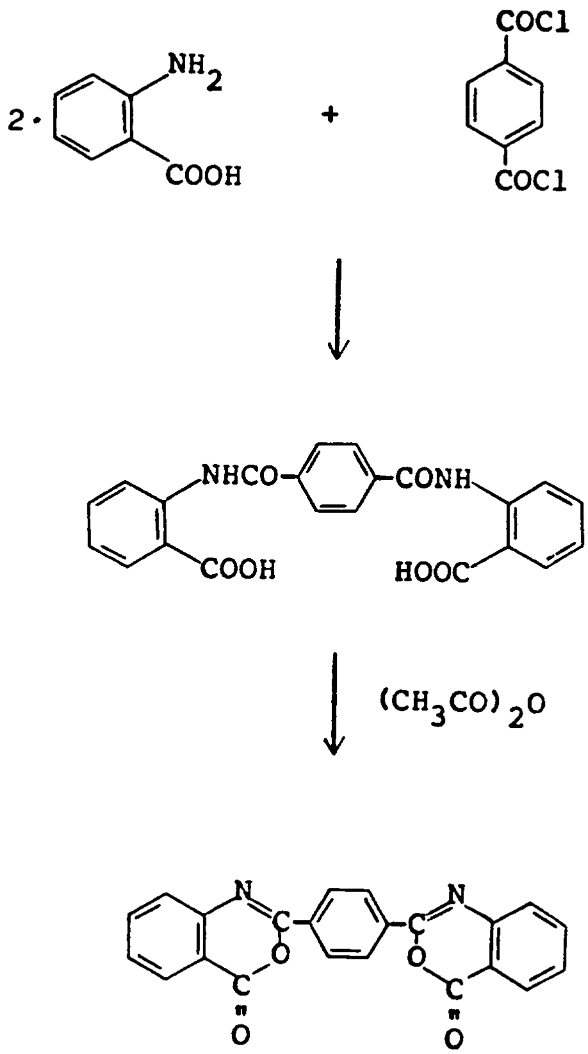


As an alternative process it is also possible to react isatoic anhydride with an aromatic dicarboxylic acid dichloride in pyridine as disclosed in DE-A-2,556,590 and the corresponding JP-A-100,086/1976.

This process can be represented by the following reaction formula when using biphenylene-4,4'-dicarboxylic acid dichloride as the aromatic dicarboxylic acid dichloride:

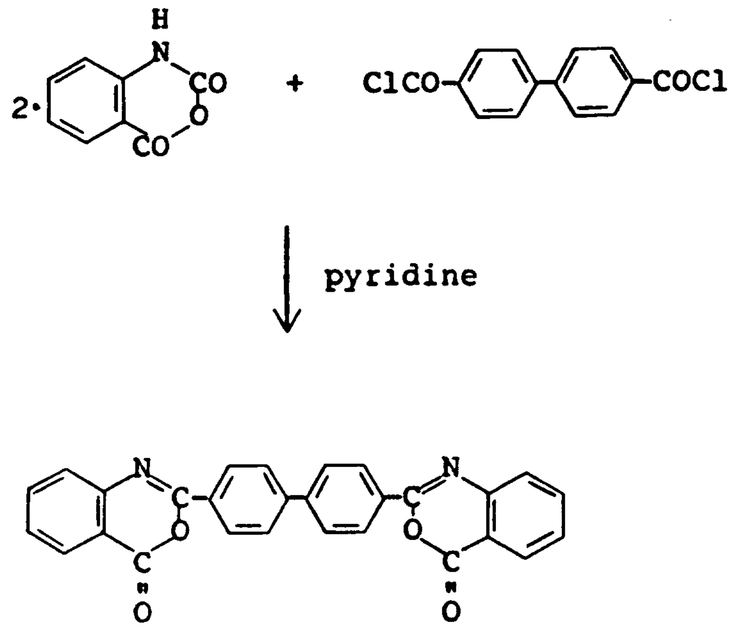


The cosmetic of the invention is not only safe when it is directly applied onto the skin of a human being, but also is colorless, odorless and non-stimulative. The cosmetic further is non-volatile, chemically stable so that it does not decompose with sweating or washing, and moreover is stable against sunlight so that it sustains its ultraviolet ray-absorbing ability over a long time.

Thus our researches have revealed that since the cyclic imino esters of formula (I) have an excellent ultraviolet ray-absorbing ability and have excellent water resistance, they provide a prolonged ultraviolet ray-absorbing ability. Moreover since these esters are sparingly soluble in organic solvents, fats and oils and sebum, they are stable compounds which are not percutaneously absorbed and moreover have a low stimulating property to the skin and can prevent aging of the skin.

The cosmetics of the invention are those wherein a compound of formula (I) is compounded into cosmetic bases. The form of the cosmetic can vary depending upon the kind of cosmetic base. Thus, it is possible to prepare a fat or oil type or non-fat or oil type ointment or skin cream by compounding with an ointment base or cream base, and also it is possible to prepare a skin care agent by mixing with a solvent. Examples of suitable bases include hydrocarbons such as solid or liquid paraffin and montan wax; fats and oils such as olive oil and lanolin; fatty acids and their esters such as stearic acid and glycerin monooleate; and alcohols such as cetyl alcohol, palmityl alcohol and glycerin.

The cosmetics of the invention can be used in the form of milky lotion, cream, aqueous milky lotion, gel or foam.

When classified by application site or use, the cosmetic of the invention can be, for example, a face cream; a make-up product such as make-up cake or foundation, make-up lotion, make-up powder, or make-up cream; a lotion or astringent for the face; a body oil or lotion; or a hand lotion or cream.

Although the quantity of the ultraviolet ray-absorbing compound of formula (I) to be compounded in the cosmetic of the invention varies depending on what extent of protective effect against ultraviolet ray is intended to be attained, it is generally desirable that the quantity is 0.2 to 20% by weight based on the weight of the whole composition. One or more compounds of formula (I) compound can be compounded, and the cosmetic of the invention can also contain a known ultraviolet ray absorber.

The cosmetic of the invention can contain usual auxiliary agents for cosmetics such as emulsifier, pigment, dye, softening agent, lubricant, surfactant, antiseptic agent, antifoaming agent and perfume, and other optional components directly used in cosmetic compositions.

The present invention is further described in more detail below by examples. Parts in the examples are by weight parts

## EMBODIMENTS

Reference example (Preparation of cyclic imino ester)

Anthranilic acid (14.0 parts) and 11.7 parts of sodium carbonate were dissolved in 250 parts of water, and a solution obtained by dissolving 10.1 parts of terephthalic acid dichloride in 60 parts of acetone was added dropwise with stirring at 20 to 30 °C. Reaction was carried out at room temperature for 2 hours after the dropwise addition and further under acetone reflux for one hour. Then, concentrated hydrochloric acid was added to make the reaction system acidic, and the precipitate was filtered and dried to obtain 19.1 parts of terephthaloyl-bisanthranilic acid.

Then, 100 parts of acetic anhydride were added to the whole quantity of the compound, and reaction was carried out under reflux of acetic anhydride for 2 hours. The reaction mixture was cooled, and the precipitate was filtered and dried to obtain 15.5 parts of 2,2'-p-phenylenebis(3,1-benzoxazin-4-one).

Further, the same procedures as above were carried out except that 4,4'-diphenyldicarboxylic acid dichloride and naphthalene-2,6-dicarboxylic acid dichloride were used respectively in place of terephthalic acid dichloride, whereby 2,2'-(4,4'-diphenylene)bis(3,1-benzoxasin-4-one) and 2,2'-(2,6-naphthylene)bis(3,1-benzoxazin-4-one) were obtained respectively.

EXAMPLE 1 (Measurement of ultraviolet ray-absorbing ability)

There were measured ultraviolet ray-absorbing ability of the cyclic imino esters used in accordance with the invention and a commercially available known ultraviolet absorber (4-t-butyl-4'-methoxy-dibenzoylmethane).

The measurement was carried out using 1,1,2,2-tetrachloroethane as a solvent and Hitachi 330 type spectrophotometer under the conditions of an ultraviolet absorber concentration of $5x10^{-4}$ g/100 ml and an optical path length of 1 cm. The results are shown in Table 1.

Table 1

| | λmax(nm), (E 1%, 1cm) | λs(nm) |
|---|---|---|
| 2,2'-p-phenylenebis (3,1-benzoxazin-4-one) | 350 (1270) | 385 |
| 2,2'-(4,4'-diphenylene)bis(3,1-benzoxazin-4-one) | 348 (1300) | 395 |
| 2,2'-(2,6-naphthylene) bis(3,1-benzoxazin-4-one) | 354 (1070) | 400 |
| (Comparative example) 4-t-butyl-4'-methoxydibenzoylmethane | 360 (1040) | 400 |
| Note: λmax and λs represent maximum absorption wavelength and absorption intiation wavelength | | |

EXAMPLE 2 (Measurement of solubility)

A cyclic imino ester and the comparative sample were assessed for solubility in water, alcohols and oily cosmetic bases at 15 °C.

The results are shown in Table 2.

Table 2

| Solubility (mg/l) | | |
|---|---|---|
| | (Present invention) 2,2'-p-phenylenebis (3,1-benzoxazin-4-one) | (Comparative example) 4-t-butyl-4'-methoxydibenzoylmethane |
| Water | 0.2 | 0.2 |
| 966 vol % Ethanol | 0.3 | $1.6 \times 10^4$ |
| Glycerin | 0.4 | $2.5 \times 10^3$ |
| Light liquid paraffin | 2 | $8.5 \times 10^3$ |
| Isopropyl myristate | 15 | $5.3 \times 10^4$ |
| Olive oil | 20 | $1.9 \times 10^4$ |
| Castor oil | 19 | $9.6 \times 10^4$ |

It can be seen from Table 2 that the cyclic iminoester has a smaller solubility in the organic solvents and thus smaller absorbability into a human body compared to the comparative sample. The cyclic iminoester is therefore safer to use.

EXAMPLE 3 (Measurement of water resistance)

Fifty milliliters each of aqueous suspensions containing 10 % by weight of a cyclic imino ester and a comparative sample, respectively was prepared, and allowed to stand in a constant temperature bath of 80 °C for 2 hours.

Then, 50 ml of isopropyl alcohol was added thereto, and the water and the solvent were removed using a rotary evaporator, and the residue was taken out.

This residue was measured for molecular extinction coefficient in λmax according to a similar manner to Example 1, and its change was used as a measure of water resistance.

$$\text{Water resistance (\%)} = \frac{\text{Molecular extinction coefficient of sample after water resistance test}}{\text{Molecular extinction coefficient of sample before water resistance test}} \times 100$$

Further, the samples before and after hot water treatment were measured for TG-DTA, and its change was examined.

The results are shown in Table 3.

Table 3

| | Water resisting stability (%) | Change of TG-DTA |
|---|---|---|
| Present invention | | |
| 2,2'-p-phenylenebis(3,1-benzoxazin-4-one) | 96.9 | no change |
| 2,2'-(4,4'-diphenylene)bis(3,1-benzoxazin-4-one) | 97.6 | same as above |
| 2,2'-(2,6-naphthylene)bis(3,1-benzoxazin-4-one) | 96.7 | same as above |
| Comparative sample | | |
| 4-t-butyl-4'-methoxy-dibenzoylmethane | 89.4 | generation of subpeak |
| 4-methoxy-2'-sodium carboxylate-dibenzoylmethane | 52.0 | same as above |

It is seen from the above Table 3 that the cyclic imino esters are superior to the comparative samples in stability against water and have a sustained ultraviolet ray-absorbing effect.

EXAMPLE 4 (Primary irritation to the skin)

This experiment was carried out according to the following method.

1) Eight white rabbits (weighing 2.0 to 3.0 kg) whose fur on the back was cut are grouped into two groups each consisting of 4 animals. Animals of one group (blank) were fixed as they were in braces and those of the other group were fixed in braces after excoriation at the site to be tested.

2) Vaseline base containing 25 % by weight of a test substance was applied onto a lint cloth, and the resulting lint cloth was stuck on the surface of skin.

3) Whole body of the rabbit was wrapped with moisture-proof rubber for dental use to prevent movement of the stuck lint cloth and at the same time evaporation of volatile substance.

4) Twenty-four hours thereafter the lint cloth was removed and the test site was observed. Second and third observations are made 48 and 72 hours thereafter, respectively. The average of evaluation after 24 and 72 hours was calculated as a final evaluation point.

5) Evaluation was made by independently judging formation of erythema and eschar, and edema, specifically according to the following criteria.

| Formation of erythema and eschar: | |
|---|---|
| No erythema | - |
| Very slight erythema (barely perceptible) | ± |
| Well defined erythema | + |
| Moderate to severe erythema | + + |
| Severe erythema (beet redness) to slight formation of eschar | + + + |

| Formation of edema | |
|---|---|
| No edema | - |
| Very slight edema (barely perceptible) | ± |
| Slight edema (edge of area well defined by definite raising) | + |
| Moderate edema (raised approximately 1 mm) | + + |
| Severe edema (raised more than 1 mm and extending beyond area of exposure) | + + + |

The results are shown in Table 4.

Table 4

| | Cyclic imino ester | | |
|---|---|---|---|
| Formation of erythema or eschar | A | B | C |
| - | 4/4 | Same as left | Same as left |
| ± | 0/4 | Same as left | Same as left |
| + | 0/4 | Same as left | Same as left |
| + + | 0/4 | Same as left | Same as left |
| + + + | 0/4 | Same as left | Same as left |
| Formation of edema | 0/4 | 0/4 | 0/4 |

A, B and C in Table 4 are as follows:

A: 2,2'-p-phenylenebis(3,1-benzoxazin-4-one)

B: 2,2'-(4,4'-diphenylene)bis(3,1-benzoxazin-4-one)

C: 2,2'-(2,6-naphthylene)bis(3,1-benzoxazin-4-one)

No formation of erythema, eschar or edema was observed for the three cyclic imino esters.

EXAMPLE 5 (Mutagenicity)

This experiment was carried out based on "About Test on Mutagenicity Using Microorganisms", a notification of the Director of the Standard Bureau, the Ministry of Labor, Japan dated May 18, 1985.

No mutagenicity was observed for any of the cyclic imino esters A, B and C used in Example 4.

EXAMPLE 6 (Allergic contact dermatitis test)

Method: This experiment was carried out as follows based on "Guinea Pig Maximization Test" (Magnusson, B. & Klingman, A. M: Usefulness of guinea pig tests for detection of contact sensitizers, In Advances in Modern Toxicology. Vol 4, pp 551-560 Hemisphere Publishing Co., Washington & London, 1977)

1) The fur on the scapula of white female guinea pigs weighing 300 to 500 g was cut in an area of 4 x 6 cm.

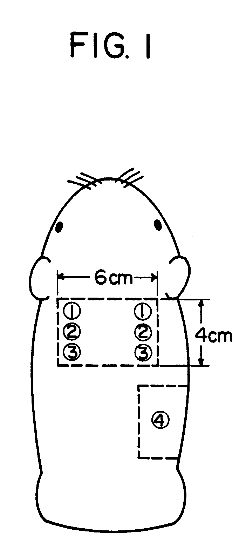

2) Each sample was intradermally injected into a guinea pig at the positions (1), (2) and (3) shown in Figure 1 with

(1) 0.1 ml of Freund's complete adjuvant alone;

(2) 0.1 ml of test substance alone; or

(3) sample w/o emulsified with 0.1 ml of Freund's complete adjuvant

3) The fur at the sites was cut one week after the intradermal injection, and a non-irritating test substance, 10 % sodium lauryl sulfate (vaseline base), was applied thereon.

4) Vaseline containing 25 % by weight of the test substance was spread on a filter paper of 2 x 4 cm and occlusive pasting was conducted for 48 hours.

5) The fur at the side of the body of a guinea pig shown at (4) in Figure 1 was cut in an area of 5 x 5 cm two weeks after the occlusive pasting.

6) The test substance was applied onto a filter paper of 2 x 2 cm, and occlusive pasting was conducted for 24 hours.

7) Measurement was carried out according to the following criterion:

| Symbol | Criterion of Judgement |
|---|---|
| 0 | No reaction |
| 1 | Light or polynesic erythema |
| 2 | Diffuse erythema in middle degree |
| 3 | Strong erythema plus edema |

Results are shown in Table 5 in case where the cyclic imino esters A, B and C used in Example 4 were used as test substance.

Table 5

| | Cyclic imino ester | | |
|---|---|---|---|
| Judgement | A | B | C |
| 0 | 4/4 | Same as left | Same as left |
| 1 | 0/4 | Same as left | Same as left |
| 2 | 0/4 | Same as left | Same as left |
| 3 | 0/4 | Same as left | Same as left |

Contact sensitization was not observed for any of these compounds.

EXAMPLE 7 (Contact photosensitization)

This experiment was conducted as follows based on Adjuvant-Strip method (Seinichi Hifu, 42 (5), 831-837, 1980).

(1) Hartley white guinea pigs weighing 370 to 420 g were used.

(2) The fur at the cervical part of guinea pigs was cut by an eletric hair clipper, and the part was further shaved with an electric razor.

(3) A section of 2 x 4 cm was set at the cervical part, and 0.1 ml portions of Freund's complete adjuvant w/o emulsified with sterilized distilled water were intradermally injected at the four corners.

(4) The skin of the section of 2 x 4 cm was subjected to stripping with cellophane tape to cause light erythema.

(5) 0.1 ml of vaseline containing 25 % by weight of the test substance was applied onto this site.

(6) After the application, 6 lamps in total, namely 3 sunlamps (Toshiba FL-40SE30) and 3 black lights (Toshiba FL-40SBLB) were arranged in parallel, 70 minutes irradiation was carried out at a distance of 10 cm from the light sources. Total energy of the irradiation at this time was 12.0 J/$cm^2$.

(7) The procedures (4), (5) and (6) were repeated 5 times every other day.

(8) Two weeks after completion of the sensitization, the fur on the whole back of guinea pig was cut, and the resulting whole back was epilated for 15 minutes with an emulsion type epilating cream containing 3.3 % of thioglycolic acid.

(9) 24 Hours after the epilation, 4 sections each of 1.5 cm x 1.5 cm were set in two rows using the medium line as a symmetry axis.

(10) This site was coated with the test substance and irradiated for 70 minutes at a distance of 10 cm from 6 black lights. Total energy at this time was 10.2 J/$cm^2$.

(11) Judgement was made 24 and 48 hours after the irradiation according to the following criterion:
[Judgement criterion]

| Formation of erythema | |
|---|---|
| No erythema | 0 |
| Erythema in very light degree | 1 |
| Distinct erythema | 2 |
| Erythema in middle to heavy degree | 3 |
| Heavy erythema wherein reaction is deep and to which slight eschar is added | 4 |

Results are shown in Table 6 in case where the cyclic imino esters A, B and C used in Example 4 were used as test substance.

Table 6

| | Cyclic imino esters | | |
|---|---|---|---|
| Judgement | A | B | C |
| Formation of erythema | | | |
| 0 | 10/10 | Same as left | 9/10 |
| 1 | 0/10 | Same as left | 1/10 |
| 2 | 0/10 | Same as left | Same as left |
| 3 | 0/10 | Same as left | Same as left |

Content photosensitization was not observed at all for the cyclic imino esters A and B. Very slight contact photosensitization was observed for C, but only after sensitisation in a degree which is utterly out of the question in practice.

<u>EXAMPLES 8 AND 9 AND COMPARATIVE EXAMPLE</u> (w/o type cream)

Cosmetics indicated in Table 7 were prepared.

Table 7

| | | Example 8 | Example 9 | Comparative example |
|---|---|---|---|---|
| A) | 2,2'-p-phenylene bis(3,1-benzoxazin-4-one) | 6 parts | - part | - part |
| | 2,2'-(4,4'-diphenylene)bis(3,1-benzoxazin-4-one) | - | 6 | - |
| | Myristic acid isopropyl ester | 10 | 10 | 10 |
| | Vaseline | 10 | 10 | 16 |
| | Salt of sulfuric ester of alcohol by reduction of tallow (sulfation rate 10 %) | 15 | 15 | 15 |
| | 4-Hydroxybenzoic acid propyl ester | 0.1 | 0.1 | 0.1 |
| B) | Glycerin | 1.5 | 1.5 | 1.5 |
| | 1,2-Propylene glycol | 1.5 | 1.5 | 1.5 |
| | Aqueous sorbitol solution | 4 | 4 | 4 |
| | Water | about 100 | about 100 | about 100 |

Compositin A) was mixed with heating to about 70°C, and added to the water layer composition B) warmed to about 70°C. After stirring, the mixture was cooled to about 40°C, perfume was added thereto and the mixture was homogenized.

Skin protection test against UV-A

This test was carried out using guinea pigs (one group 7 animals) based on the method of Nakayama, et al. (Sunlight and Man page 59, published by Todai Shuppan, 1974).

Previously, 8 mg/kg of 8-methoxypsoralen was orally administered to the guinea pigs to enhance sensitivity to UV-A.

Creams of Examples 8 and 9 and Comparative example were applied onto the skin which was shaved on the back, and effects in the examples were compared to those in case of non-application and in case of Comparative example using as a light source Toshiba FL-40BLB lamp (λmax 360 nm).

The results are shown in Table 8. Each value means number of animals in which erythema was formed at each irradiation time.

Table 8

| Irradiation time (min.) | Example 8 | Example 9 | Comparative example | Non-application |
|---|---|---|---|---|
| 4 | 0 | 0 | 0 | 2 |
| 6 | 0 | 0 | 1 | 7 |
| 8 | 0 | 0 | 3 | 7 |
| 10 | 0 | 0 | 5 | 7 |
| 15 | 0 | 0 | 7 | 7 |
| 20 | 0 | 0 | 7 | 7 |
| 30 | 0 | 0 | 7 | 7 |

Table 8 indicates that Examples 8 and 9 perfectly protect the skin from UV-A compared to the case of non-application and Comparative example and thus have an excellent effect as a cosmetic for prevention of sunburn.

EXAMPLE 10 (o/w type cream)

| | | |
|---|---|---|
| A) | 2,2'-p-phenylenebis(3,1-benzoxazin-4-one)<br>Saturated fatty acid triglyceride having 8 to 10 carbon atoms Cetyl alcohol<br>Emulsified mixture of stearyl alcohol, cetyl alcohol and their ethylene oxide adducts<br>4-Hydroxybenzoic acid propyl ester | 8 parts<br>10 1.5<br>8 parts<br>0.1 |
| B) | Glycerin<br>1,2-Propylene glycol<br>Aqueous sorbitol solution<br>Water | 1.5<br>1.5<br>4<br>about 100 |

Composition A) was mixed with warming to about 70 °C, the mixture was added to the water layer composition B) warmed to about 70 °C followed by mixing with stirring, and the resulting mixture was cooled to about 40 °C and homogenized after addition of perfume.

EXAMPLE 11 (Emulsion)

| | |
|---|---|
| 2,2'-(2,6-naphthylene)bis(3,1-benzoxazin-4-one) | 10 parts |
| Gossypol wax (rice bran oil wax) | 5 |
| Vaselin oil | 6 |
| Myristic acid isopropyl ester | 3 |
| 4-Hydroxybenzoic acid propyl ester | 0.3 |
| Glycerin | 20 |
| Water | about 56 |

The above composition was mixed with stirring, and after addition of perfume the mixture was homogenized.

EXAMPLE 12 (Oil foundation)

| | |
|---|---|
| Liquid paraffin | 15 parts |
| 2,2'-p-phenylenebis(3,1-benzoxazin-4-one) | 15 |
| Palmitic acid isopropyl ester | 10 |
| Lanolin alcohol | 3 |
| Microcrystalline wax | 7 |
| Candelilla wax | 0.5 |
| Ozocerite | 8 |
| Titanium oxide | 8 |
| Kaolin | 18 |
| Talc | 6 |
| Red iron oxide | 1.5 |
| Black iron oxide | 0.5 |
| 4-Hydroxybenzoic acid propyl ester | 0.3 |

The above composition was mixed with stirring, and after addition of perfume the mixture was homogenized.

## Claims

1. A cosmetic containing a cyclic imino ester of formula (I)

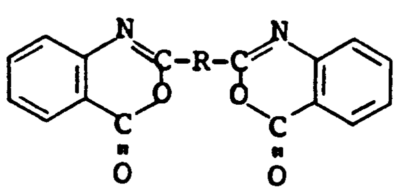

... (I)

(wherein R is a divalent aromatic hydrocarbon residue) in a quantity effective for the absorption of ultraviolet radiation, together with cosmetic base.

2. A cosmetic according to claim 1 wherein R is phenylene, biphenylene or naphthylene.

3. A cosmetic of claim 1 which is in the form of a cream, foundation, powder, stick or oil.

4. A method for protecting a site on a human body against ultraviolet radiation which comprises applying thereto an effective amount of a cosmetic as claimed in claim 1, 2 or 3, wherein treatments of the

human body by surgery or therapy and diagnostic methods practised on the human body are excluded.

**5.** Use of a cyclic imino ester of formula (I) as defined in claim 1 as an ultraviolet absorber in a cosmetic.

## Patentansprüche

**1.** Kosmetikum, enthaltend einen cyclischen Iminoester der Formel (I)

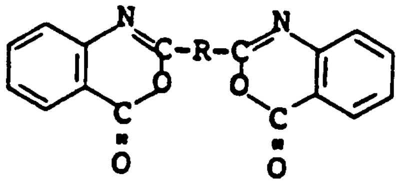

. . . (I)

(in der R ein zweiwertiger aromatischer Kohlenwasserstoffrest ist) in einer Menge, die wirksam ist zur Absorption von UV-Strahlung, zusammen mit einer kosmetischen Grundsubstanz.

**2.** Kosmetikum nach Anspruch 1, wobei R Phenylen, Biphenylen oder Naphthylen ist.

**3.** Kosmetikum nach Anspruch 1 in Form einer Creme, einer Unterlage, eines Pulvers, eines Stifts oder eines Öls.

**4.** Verfahren zum Schutz einer Stelle des menschlichen Körpers gegen UV-Strahlung, umfassend das Aufbringen einer wirksamen Menge eines Kosmetikums nach Anspruch 1, 2 oder 3, wobei die Behandlung des menschlichen Körpers durch medizinische oder therapeutische und diagnostische Verfahren, die am menschlichen Körper durchgeführt werden, ausgeschlossen ist.

**5.** Verwendung eines cyclischen Iminoesters der Formel (I), wie in Anspruch 1 definiert, als UV-Absorber in einem Kosmetikum.

## Revendications

**1.** Cosmétique contenant un imino ester cyclique de formule (I)

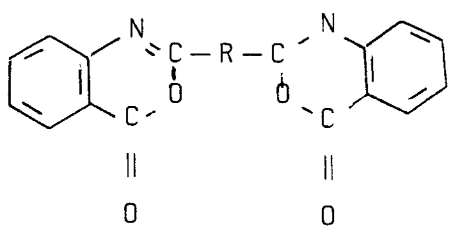

(dans laquelle R est un radical hydrocarboné aromatique divalent), en une quantité effective pour absorber les rayons ultraviolets, ainsi qu'une base cosmétique.

**2.** Cosmétique selon la revendication 1, caractérisée en ce que R est phénylène, biphénylène ou napthylène.

**3.** Cosmétique selon la revendication 1, caractérisée en ce qu'elle se présente sous forme de crème, de base de maquillage, de poudre, de stick ou d'huile.

**4.** Procédé de protection d'une partie du corps humain contre les rayons ultraviolets , caractérisé en ce qu'il comprend l'application sur cette partie d'une quantité effective d'un cosmétique tel que revendiqué

dans les revendications 1, 2 ou 3, dans lequel les traitements du corps humain par chirurgie ou thérapie et méthodes de diagnostic pratiqués sur le corps humain sont exclus.

**5.** Utilisation d'un imino ester cyclique de formule (I) telle que définie dans la revendication 1 à titre d'absorbant ultraviolet dans un cosmétique.

FIG. 1